# EUROPEAN PATENT APPLICATION

(11) **EP 4 852 017 A1**
(43) Date of publication of application: **09.12.2026**
(21) Application number: 25749021.9
(22) Date of filing: 24.01.2025
(51) Int. Cl.: G16H 50/70, G16H 30/20, G16H 50/50, G16H 80/00, G06F 3/0482, G06F 3/04817, G16H 10/60, A61B 5/318, A61B 6/50, G06N 3/0464

(54) **DEVICE AND METHOD FOR ANALYZING MEDICAL IMAGE**

(30) Priority: 30.01.2024 KR 20240014325
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2025/001491
(87) International publication number: WO 2025/165118

(57) **Abstract**

A device for analyzing a medical image by using artificial intelligence according to an embodiment: inputs a medical image to a first model to extract a feature vector that a second model uses to perform a first task; inputs the feature vector to the second model to cause the second model to perform the task; provides a first medical result describing a health state based on the medical image on the basis of an output result of the second model; and performs a second task requested by a user of the device for the first medical result, wherein the second task comprises: a first additional task of transferring the first medical result to the outside; and a second additional task of performing secondary analysis including at least one of assisted decision-making and difference analysis on the first medical result.

## Description

### [Technical Field]

The disclosed embodiments relate to a technology for analyzing a medical image by using artificial intelligence and for supporting a user in making a decision based on a result of the analysis.

### [CROSS-REFERENCE TO RELATED APPLICATION]

This application claims priority to Korean Provisional Patent Application No. 10-2024-0014325, filed on January 30, 2024, the entire disclosure of which is incorporated herein by reference.

### [Background Art]

In the medical field, artificial intelligence technology is actively used. For example, a doctor evaluates a patient's health state by utilizing artificial intelligence. Recently, with the miniaturization of terminals, such artificial intelligence technology is also implemented in personalized devices such as smartphones. Therefore, services in which doctors analyze a patient's examination results using personalized devices have been developed and are widely used.

In such utilization on a personalized device, once an analysis result is provided to a corresponding user, additional utilization thereof is impossible. Because artificial intelligence analysis consumes expensive resources, such inefficient utilization should be avoided. In other words, there is a need for a solution by which a result of a single examination act can be shared among a plurality of medical staff members, and by which the result can be additionally analyzed and utilized secondarily according to a purpose.

### [Disclosure]

### [Technical Problem]

The disclosed embodiments are intended to analyze a medical image by using artificial intelligence and to support a user in making a medical-based decision based on a result of the analysis.

### [Technical Solution]

A device for analyzing a medical image by using artificial intelligence according to an embodiment is a device for analyzing a medical image by using artificial intelligence, the device including: one or more processors; and a memory storing instructions executed by the one or more processors, wherein the one or more processors are configured to: input the medical image to a first model to extract a feature vector that a second model uses to perform a first task; input the feature vector to the second model to cause the second model to perform the first task; provide a first medical result describing a health state of the medical image based on an output result of the second model; and perform a second task requested by a user of the device with respect to the first medical result, wherein the second task includes: a first additional task of transferring the first medical result to the outside; and/or a second additional task of performing a secondary analysis including at least one of assisted decision-making and difference analysis on the first medical result.

The medical image may include at least one of an electrocardiogram image and a chest radiographic image acquired by the device.

The one or more processors may be configured to: when a request to transfer the first medical result to the outside is received from the user, display, on a screen of the device, information of one or more other users to whom the transfer is possible, the information of the one or more other users including at least one of a department and a medical institution to which the other user belongs; and transfer the first medical result to a specific user selected by the user from among the one or more other users.

The one or more processors may be configured to: record a transfer history of the first medical result between the user and the specific user in a consultation channel in which the user and the specific user participate; and display, on a preview screen of the consultation channel, at least one of a department to which the other user belongs, the medical institution to which the other user belongs, a last conversation content of the consultation channel, and a last conversation time.

The one or more processors may be configured to display the first medical result on a background screen in the consultation channel.

The one or more processors may be configured to display, on a screen of the device, all medical results including the first medical result and another medical result previously acquired by the device, together with whether output has succeeded, in a list, wherein the other medical result may include a medical result that the device previously acquired by inputting another medical image to the first model and the second model before inputting the medical image to the first model.

The one or more processors may be configured to: analyze, for some medical results, selected by the user and including the first medical result, from among all the medical results, a difference with respect to the first medical result based on a selection order of the user; and provide the difference visually based on the selection order.

The one or more processors may be configured to display, together with a list of the some medical results, a number corresponding to the selection order of the user.

The one or more processors may be configured to provide, as the first medical result, at least one of a rhythm type of a heart, a contractility, a value and distribution of a biomarker, a sensitivity, and a health-related recommendation corresponding to the medical image.

The one or more processors may be configured to input an original image of the medical image to a third model to process the original image into the medical image, wherein the third model may be pre-trained to, when an input image is input, classify an examination type of the input image and process the input image according to a region of interest that is set corresponding to the examination type.

The one or more processors may be configured to, when a click event by the user occurs on a first user interface displayed on a screen of the device, call a function linked to the click event, and through the function, remove the first user interface from the screen and display a second user interface on the screen.

The second task may include a secondary analysis function accessible by the device through purchase in an app store in which one or more web services are registered, and the secondary analysis function may be executed when a click event occurs on the second user interface.

A method for analyzing a medical image by using artificial intelligence performed by a device including one or more processors and a memory storing instructions executed by the one or more processors, the method including: inputting the medical image to a first model to extract a feature vector that a second model uses to perform a first task; inputting the feature vector to the second model to cause the second model to perform the first task; providing a first medical result describing a health state of the medical image based on an output result of the second model; and performing a second task requested by a user of the device with respect to the first medical result, wherein the performing of the second task includes: performing a first additional task of transferring the first medical result to the outside; and/or performing a second additional task of performing a secondary analysis including at least one of assisted decision-making and difference analysis on the first medical result.

The medical image may include at least one of an electrocardiogram image and a chest radiographic image acquired by the device.

The performing of the first additional task may include: when a request to transfer the first medical result to the outside is received from the user, displaying, on a screen of the device, information of one or more other users to whom the transfer is possible, the information of the one or more other users including at least one of a department and a medical institution to which the other user belongs; and transferring the first medical result to a specific user selected by the user from among the one or more other users.

The performing of the first additional task may include: recording a transfer history of the first medical result between the user and the specific user in a consultation channel in which the user and the specific user participate; and displaying, on a preview screen of the consultation channel, at least one of a department to which the other user belongs, the medical institution to which the other user belongs, a last conversation content of the consultation channel, and a last conversation time.

The performing of the first additional task may include displaying the first medical result on a background screen in the consultation channel.

The performing of the first additional task may include displaying, on a screen of the device, all medical results including the first medical result and another medical result previously acquired by the device, together with whether output has succeeded, in a list, wherein the other medical result may include a medical result that the device previously acquired by inputting another medical image to the first model and the second model before inputting the medical image to the first model.

The performing of the second additional task may include: analyzing, for some medical results, selected by the user and including the first medical result, from among all the medical results, a difference with respect to the first medical result based on a selection order of the user; and providing the difference visually based on the selection order.

The performing of the second additional task may include displaying, together with the list of the some medical results, a number corresponding to the selection order of the user.

The providing of the first medical result includes providing, as the first medical result, at least one of a rhythm type of a heart, a contractility, a value and distribution of a biomarker, a sensitivity, and a health-related recommendation corresponding to the medical image.

The method may further include inputting an original image of the medical image to a third model to process the original image into the medical image, wherein the third model may be pre-trained to, when an input image is input, classify an examination type of the input image and process the input image according to a region of interest that is set corresponding to the examination type.

The method may further include: when a click event by the user occurs on a first user interface displayed on a screen of the device, calling a function linked to the click event; and through the function, removing the first user interface from the screen and displaying a second user interface on the screen.

The second task may include an additional analysis function accessible by the device through purchase in an app store in which one or more web services are registered, and the additional analysis function may be executed when a click event occurs on the second user interface.

### [Advantageous Effects]

The disclosed embodiments can provide a medical result to a user without limitation of time and place, in that an input medical image is analyzed by using artificial intelligence to provide the medical result.

The disclosed embodiments can prevent waste of resources caused by redundantly processing the same task, in that a function of transferring a medical result analyzed by one user to another user is provided.

The disclosed embodiments can suggest a medical-based decision to a user, in that a secondary analysis including a clinical decision support system (CDSS) and difference analysis is provided in addition to a primary analysis of a medical image.

### [Description of Drawings]

FIG. 1 is a block diagram for explaining a device for analyzing a medical image according to an embodiment.
FIG. 2 is an exemplary diagram for explaining a process of inputting a medical image.
FIGS. 3A to 3E are exemplary diagrams for explaining a sharing request process and a result thereof.
FIGS. 4A to 4D are exemplary diagrams for explaining a display screen of a first medical result.
FIGS. 5A to 5D are exemplary diagrams for explaining a secondary analysis request process and a result thereof as a second task.
FIG. 6 is an exemplary diagram for explaining a user interface displayed on a screen of the device.
FIG. 7 is a flowchart for explaining a method for analyzing a medical image according to an embodiment.

### [Modes of the Invention]

The terms used herein may vary depending on the intention of a user or an operator, or on practice, in consideration of the functions used in the invention. Therefore, the definitions thereof should be made based on the content throughout this specification. The terms used in the detailed description are merely for describing embodiments and should by no means be construed as limiting. Unless clearly used otherwise, a singular expression includes the meaning of a plural expression. In this description, expressions such as "including" or "provided with" are intended to designate certain components, numbers, steps, operations, elements, or a part or combination thereof, and should not be construed as excluding the presence or possibility of one or more other components, numbers, steps, operations, elements, or a part or combination thereof, other than those described.

Although terms such as first, second, and the like are used to describe various components, these components are, of course, not limited by these terms. These terms are used only to distinguish one component from another. Accordingly, a first component mentioned below may also be a second component within the technical spirit of the present invention.

In addition, the embodiments described herein may have aspects that are entirely hardware, partially hardware and partially software, or entirely software. In this specification, "unit," "apparatus," "module," "device," "server," or "system," and the like refer to a computer-related entity such as hardware, a combination of hardware and software, or software. For example, a unit, an apparatus, a module, a device, a server, or a system may refer to hardware constituting a part or all of a platform and/or software such as an application for driving the hardware. As a specific example, a unit, an apparatus, a module, a device, a server, or a system may be implemented by a processor.

The embodiments described herein may have aspects that are entirely hardware, partially hardware and partially software, or entirely software.

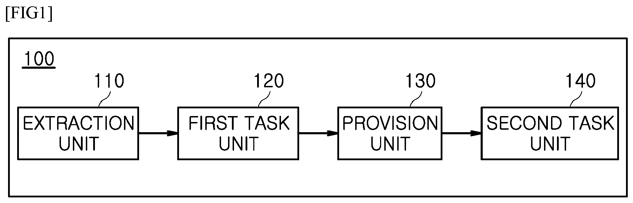

FIG. 1 is a block diagram for explaining a device for analyzing a medical image according to an embodiment.

Referring to FIG. 1, the device for analyzing a medical image includes an extraction unit, a first task unit, a provision unit, and a second task unit.

The extraction unit inputs a medical image to a first model to extract a feature vector that a second model uses to perform a first task.

The medical image may be one of various medical images acquired regardless of channel, such as black-and-white and color images, and may include, for example, at least one of an electrocardiogram image and a chest radiographic image.

Meanwhile, the medical image may be a preprocessed image. For example, the medical image may be an image in which an original image is preprocessed to fit a required size and ratio, an image already cropped from an original image into a region of interest, an image for which verification of whether it is an image related to a medical image (e.g., a 12-lead image) has already been completed, or an image in which a quality value related to noise of an input image has already been ensured.

Meanwhile, the above-described types of preprocessing are merely examples, and any processing procedure that may be performed to improve the accuracy of the first model may be included, and the preprocessing is not necessarily limited to the above-described examples.

The feature vector is medical data that can be extracted from the medical image, and may include, for example, a value related to an electrocardiogram rhythm, physiological and anatomical values, and a value related to a risk of a disease.

The first model may be a vision-based deep learning model trained to extract the feature vector from the medical image, and may be, for example, a model designed based on at least one of modified convolutional neural networks (CNNs), EfficientNet, ResNext, and Transformer.

The first task unit inputs the feature vector to the second model to cause the second model to perform the task.

Here, the second model may be a model trained to perform various tasks such as regression, classification, or event prediction based on the input feature vector.

Specifically, the second model may be a model trained to predict at least one of a rhythm type of a heart, a contractility, a value and distribution of a biomarker, a sensitivity, and a health-related recommendation based on the feature vector output by the first model.

The provision unit provides a first medical result describing a health state of the medical image based on the output result of the second model. In this case, the first medical result is a result report organized to be easily interpreted by the user, and may include at least one of a rhythm type, a contractility, a value and distribution of a biomarker, a sensitivity, and a health-related recommendation.

The second task unit performs a second task requested by a user of the device with respect to the first medical result.

The second task may include a first additional task of transferring the first medical result to the outside.

Specifically, the first additional task may include a task of processing a request by which the user intends to transfer the first medical result to the outside (e.g., to another user).

In this case, the second task unit may display information of other users on the screen of the device, for example, as a list, and, when the user who has checked the list selects a specific user, the second task unit may transfer the first medical result to the selected specific user.

Meanwhile, the list may include a medical institution and a department to which the other user belongs. Through this, the user can be provided with information that helps the user transfer the first medical result to an appropriate medical staff member.

Here, the second task unit may record the corresponding transfer history in a consultation channel in which the user and the other user participate. The second task unit may display the first medical result on a background screen of a chat room. In addition, the second task unit may display, on a preview screen of the consultation channel, at least one of a department to which the other user belongs, a medical institution to which the other user belongs, a last conversation content of the consultation channel, and a last conversation time.

The second task may include a second additional task of performing a secondary analysis including at least one of assisted decision-making and difference analysis on the first medical result.

The second task unit may provide assisted decision-making through additional analysis of the first medical result.

Here, the assisted decision-making refers to information that helps support a clinical decision based on the CDSS, and may include information useful in a process of patient diagnosis and treatment. Specifically, the assisted decision-making may include a recommendation, a warning, and a guideline appropriate for a state of the user.

The second task unit may analyze a difference with respect to the first medical result by comparing the first medical result with another first medical result.

Here, the other first medical result may be information generated by inputting another medical image to the first model and the second model before the first medical result is generated, and may refer to a past medical result for the same user that the device previously acquired.

The second task unit may display a plurality of first medical results on the screen of the device in a list form in a time order in which the first medical results were analyzed, based on a task history.

The second task unit may display, on the screen of the device, all medical results including the first medical result and another medical result previously acquired by the device in the corresponding list together with whether output has succeeded. The second task unit may also display a number corresponding to the selection order of the user together with the list of some medical results.

The second task unit may analyze, for some medical results, selected by the user and including the first medical result, from among all the medical results, a difference with respect to the first medical result based on the selection order of the user. The second task unit may provide the difference visually based on the selection order.

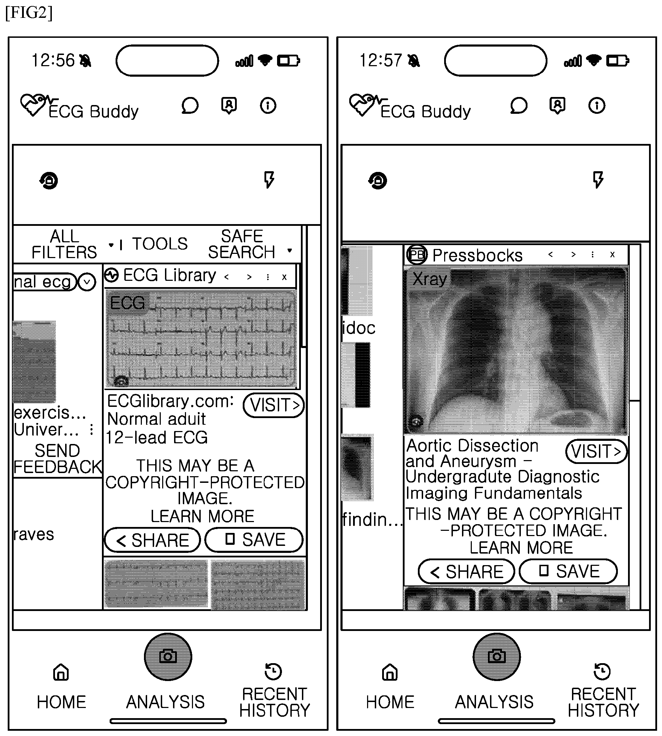

FIG. 2 is an exemplary diagram for explaining a process of inputting a medical image.

Referring to FIG. 2, the screen of the device includes a medical image. Here, the medical image is an image provided by the user or pre-stored in the device and selected by the user as an analysis target, and may be an electrocardiogram or chest radiographic image.

The medical image may be an original image selected by the user, or an image processed by automatically detecting a region of interest (e.g., a region related to an electrocardiogram or a part related to a chest radiograph) from the original image.

Specifically, the device may automatically detect the region of interest of the input image by using a third model. For example, when the device receives an original image of a medical image as input, the device first inputs the original image to the third model to acquire a medical image processed such that only the region of interest remains from the original image.

Here, the third model may be a model trained to, when an input image is input, classify an examination type of the input image and process the input image according to a region of interest that is set corresponding to the examination type. For example, the third model may classify the examination type of the input image, such as whether the input image is a chest radiographic image captured by X-ray or an electrocardiogram image representing lead waveforms. Here, the third model may also classify the examination type of the input image by learning a pattern of medical data appearing in the input image. Thereafter, the third model may automatically extract a region of interest set according to the examination type and process the corresponding region of interest into a medical image. As a specific example, the third model may automatically detect the region of interest by extracting an object in a box form from the input image, such as a CNN-based detection model, You Only Look Once (YOLO), or faster region-based CNN (Faster R-CNN).

Meanwhile, the region of interest may be automatically detected, and may also be manually set based on some points (two points or four points) selected by the user.

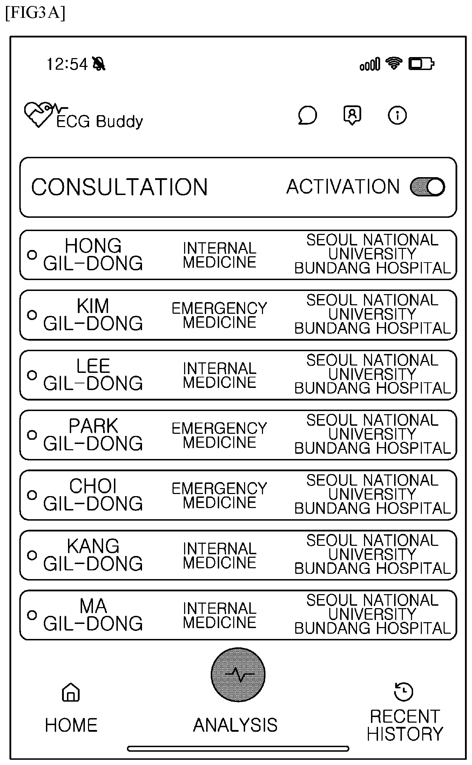

FIGS. 3A to 3D are exemplary diagrams for explaining a sharing request process and a result thereof.

Referring to FIG. 3A, information of other users to whom the first medical result may be transmitted is shown as a list on the screen of the device.

The user may identify an activation state through an activation button. For example, when an activation button corresponding to a user is activated, the corresponding user is identified as being in a state capable of replying to a consultation in the consultation channel.

A user whose activation button is activated may have personal information displayed together in the list; for example, whether the user's affiliated institution is "internal medicine" or "emergency medicine," and whether the affiliated hospital is "Seoul National University Bundang Hospital," and the like, may be displayed together in the list.

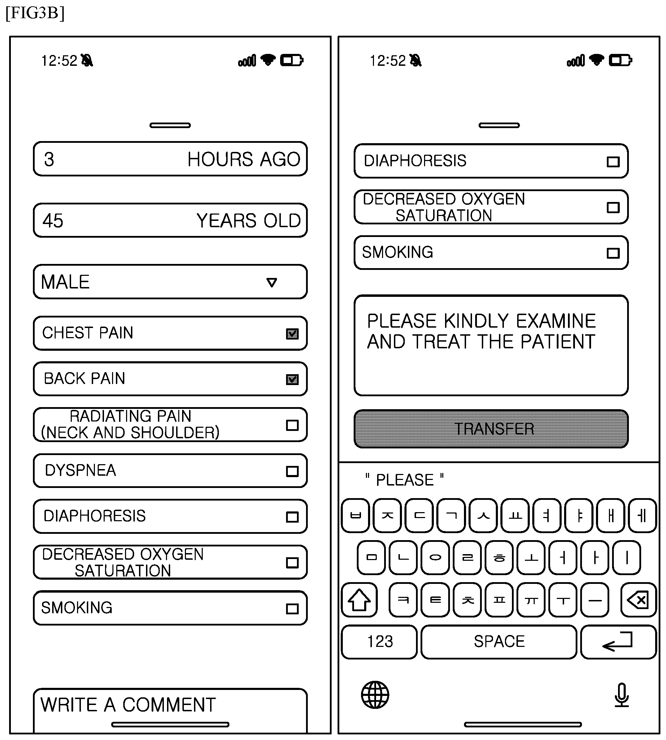

Referring to FIG. 3B, items that may be entered together with the transfer request of the user are shown.

For example, when requesting to transfer the first medical result to a specific user, the user may request that additional information selected by the user be transferred together.

For example, the user may request that at least one of a time at which the first medical result was captured, an age of a patient, a sex, a pain type, and other opinions be transferred to another user together with the first medical result.

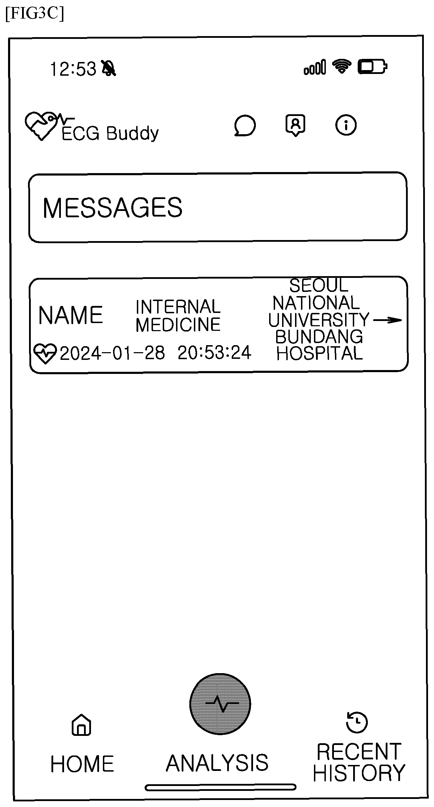

Referring to FIG. 3C, a preview screen of the consultation channel is shown.

Here, the preview of the consultation channel is a preview screen of the consultation channel that is shown after the transfer request of FIG. 3B is completed. On the preview screen of the consultation channel, information about a time at which the transfer request has been completed and about the other user (e.g., a consultant who may reply to the consultation) who has received the first medical result may be displayed.

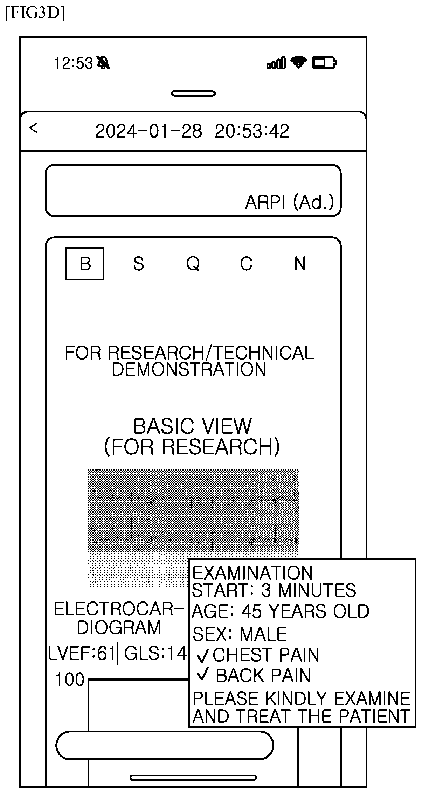

Referring to FIG. 3D, a screen of the consultation channel is shown. On the screen of the consultation channel, the first medical result exchanged between the user (e.g., a client requesting a consultation) and the other user (e.g., a consultant who has replied to the consultation) may be displayed on a background screen of the consultation channel.

From this, the user (e.g., the client requesting the consultation) may consult on the consultation while simultaneously checking the first medical result shared from the other user (e.g., the consultant who has replied to the consultation).

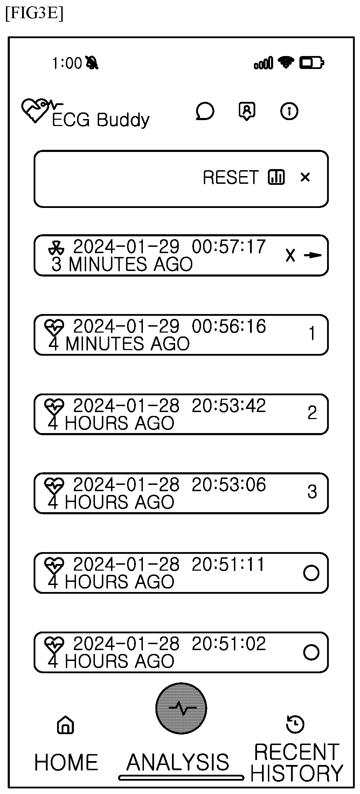

Referring to FIG. 3E, a list of medical results is displayed on the screen of the device.

On the screen, a number corresponding to an order in which the user requested and selected the medical results may be displayed together with the list of some medical results. In addition, whether reception of the medical results has succeeded may also be displayed on the screen. When reception of a medical result fails, a symbol identifying whether reception has occurred, such as "x," may be displayed together in the list.

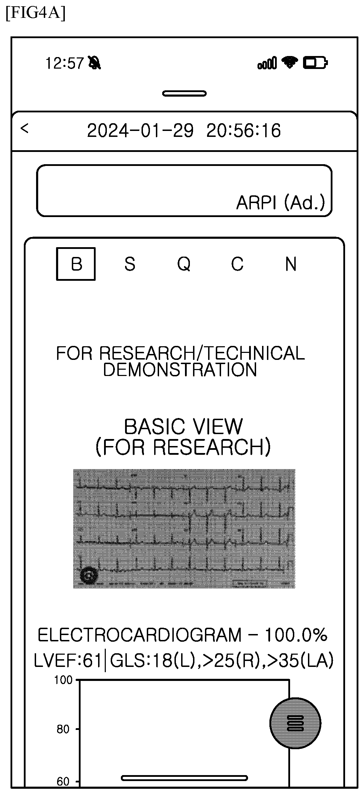

FIGS. 4A to 4D are exemplary diagrams for explaining a display screen of the first medical result.

Referring to FIG. 4A, the device may display the first medical result on the screen of the device while also displaying other advertisement information together on the screen. In this case, the device may display an advertisement related to the first medical result on the screen together with the first medical result.

The device describes the analysis of the first medical result page by page. Here, on page B, which is the first page, as illustrated, the medical image selected by the user as the analysis target may be displayed.

For example, the device may display, as the first medical result, a rhythm type (e.g., a sinus rhythm) and a contractility (e.g., a left ventricular ejection fraction, and a global longitudinal strain (GLS) of the left ventricle, the right ventricle, and the left atrium) corresponding to the medical image. In addition, the device may display a biomarker corresponding to the medical image and a numerical value thereof together on the screen.

Meanwhile, when the medical image is touched for a long time, the device may display the medical image filling the screen. In this case, the device may appropriately change a display state (horizontal or vertical) of the medical image in consideration of the size of the screen.

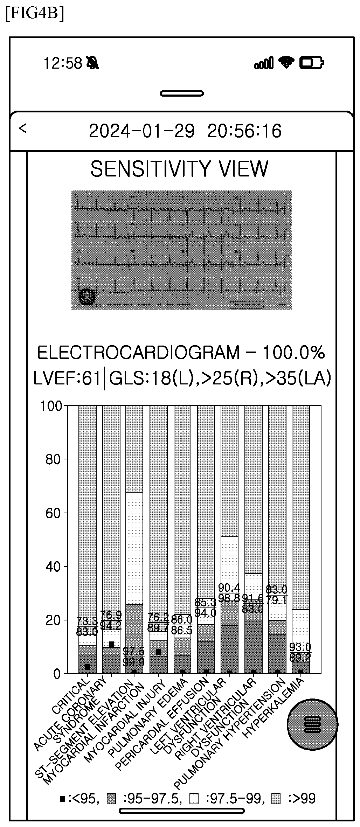

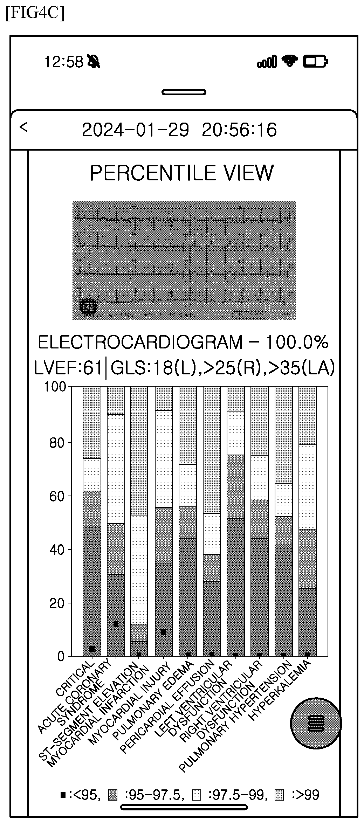

Referring to FIG. 4B, as illustrated on page S, which is the second page, the device may divide expected sensitivities according to specific threshold values over an entire biomarker section into four grades, and display, as a background, the expected sensitivity for each corresponding grade, an optimal threshold value utilizing the Youden's Index and the like, and a sensitivity and a specificity at that time. Referring to FIG. 4C, as illustrated on page Q, which is the third page, the device may visually display a biomarker distribution of a specific population as a background.

Distribution information of a biomarker corresponding to the medical image may be measured by measuring the biomarker in a specific patient group. Based on the distribution information of the biomarker, quartile sections of the biomarker may be calculated, and each section may be shown as a background in a chart in which the sections are distinguished by different colors.

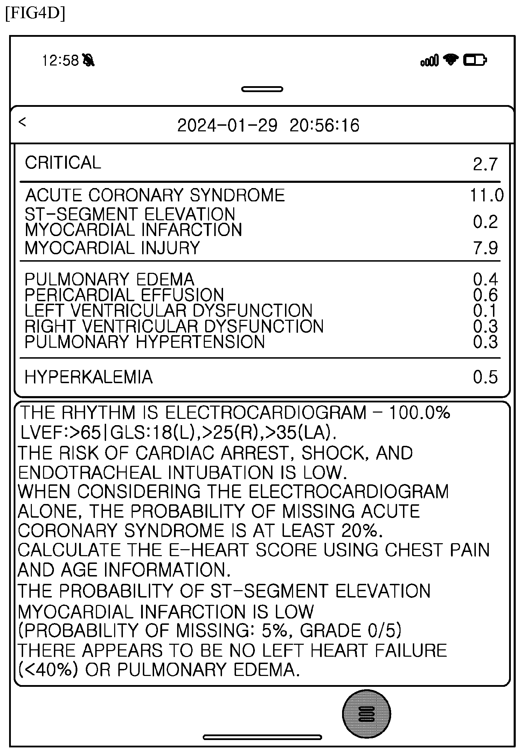

Referring to FIG. 4D, the device may display a numerical value of the biomarker in detail on the screen, and may display a preset medical recommendation based on a combination of numerical values of the biomarker.

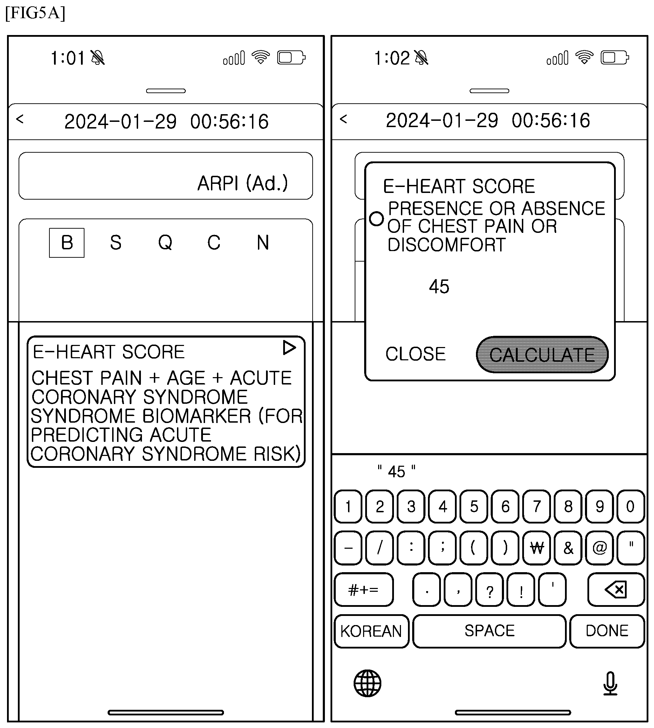

FIGS. 5A to 5D are exemplary diagrams for explaining a secondary analysis request process and a result thereof as the second task.

Referring to FIG. 5A, the device may predict assisted decision-making based on additional information on the first medical result, based on a request of the user. In this case, the additional information may include clinical information input by the user.

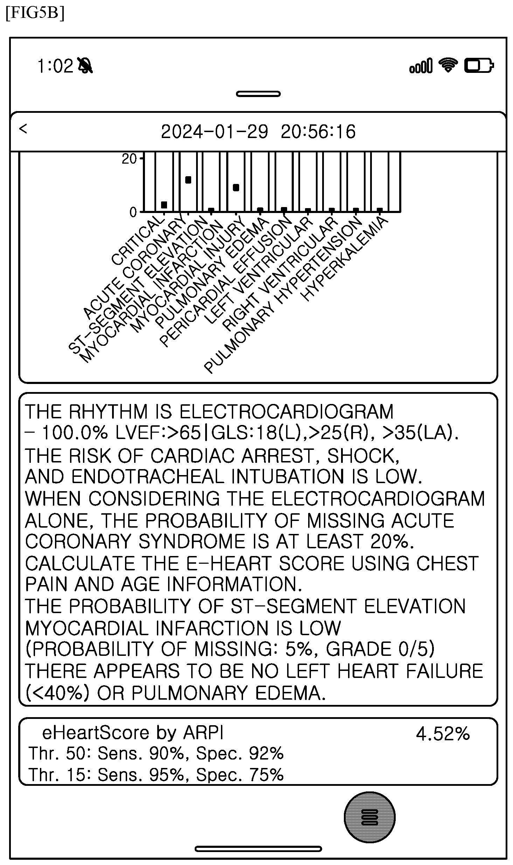

Referring to FIG. 5B, it can be seen that the assisted decision-making is displayed together with the first medical result on the screen of the device.

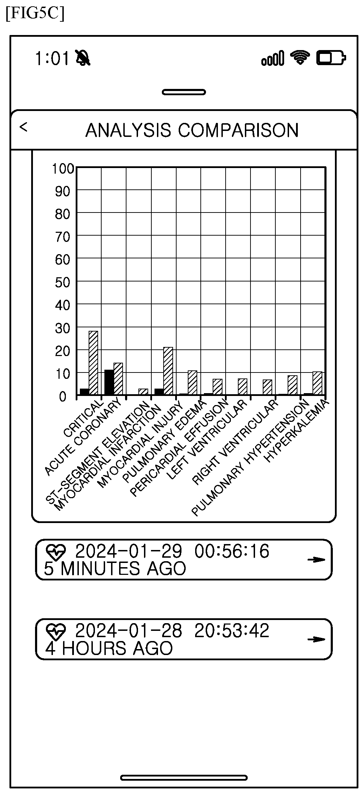

Referring to FIG. 5C, the device shows a trend of the first medical result according to a request of the user. In this case, for the first medical result, the device compares a plurality of first medical results and visually provides a difference among the first medical results.

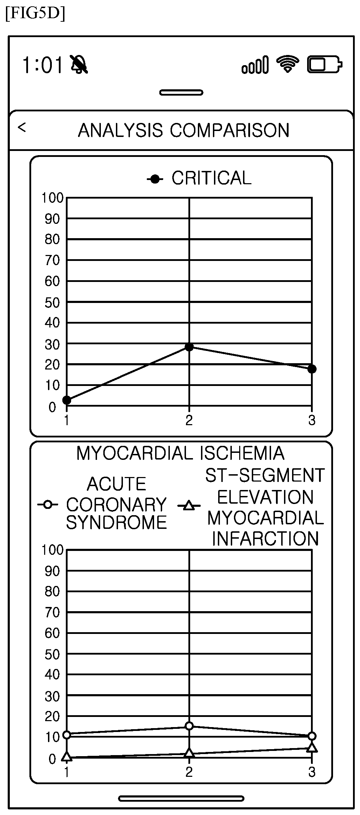

Referring to FIG. 5D, the device may compare a plurality of first medical results acquired in advance and visually provide a difference among the first medical results. Here, a number located on an x-axis of the graph refers to an analysis order selected by the user as an analysis target. For example, 1 is a symbol assigned to identify the medical result that the user first set as the analysis target, 2 is a symbol assigned to identify the medical result that the user set as the analysis target second, and 3 is a symbol assigned to identify the medical result that the user set as the analysis target third, and here, one of the medical results preferably includes the first medical result.

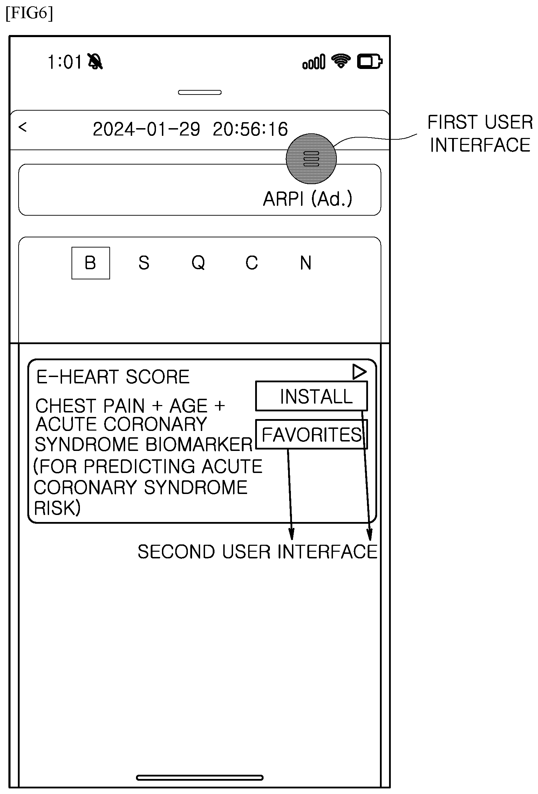

FIG. 6 is an exemplary diagram for explaining a user interface displayed on the screen of the device.

As illustrated in FIG. 6, a first user interface displayed on the screen of the device is displayed, and, when a click event on the first user interface occurs by the user, the device may call a function linked to the click event.

The device may, through the function, remove the first user interface from the screen and display a second user interface on the screen. In this case, the first user interface may be a quick menu in a drop-down menu form that shows, as a list, functions that the device may process. The second user interface may include a menu for functions that the device may process, for example, a "purchase menu," a "favorites menu," or a menu for additional analysis functions that the device may process by purchasing through the app store.

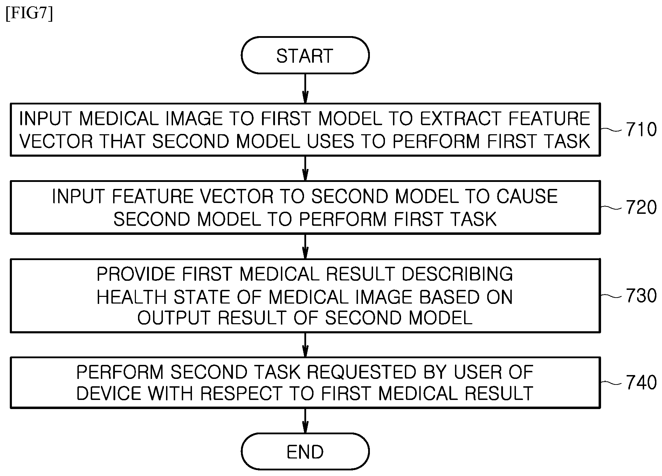

FIG. 7 is a flowchart for explaining a method for analyzing a medical image according to an embodiment.

Referring to FIG. 7, the method may be performed by the device of FIG. 1.

First, the device for analyzing a medical image according to an embodiment inputs a medical image to a first model to extract a feature vector that a second model uses to perform a first task.

Thereafter, the device for analyzing a medical image according to an embodiment inputs the feature vector to the second model to cause the second model to perform the first task.

Thereafter, the device for analyzing a medical image according to an embodiment provides a first medical result describing a health state of the medical image based on the output result of the second model.

Thereafter, the device for analyzing a medical image according to an embodiment performs a second task requested by a user of the device with respect to the first medical result.

Here, the second task includes a first additional task of transferring the first medical result to the outside; and a second additional task of performing a secondary analysis including at least one of assisted decision-making and difference analysis on the first medical result.

Meanwhile, although the method of FIG. 7 has been described as being divided into a plurality of steps, at least some of the steps may be performed in a changed order, performed together in combination with other steps, omitted, performed by being divided into sub-steps, or performed by adding one or more steps not illustrated.

Although representative embodiments of the present invention have been described in detail above, those of ordinary skill in the art to which the present invention pertains will understand that various modifications are possible with respect to the above-described embodiments within the scope of the present invention. Therefore, the scope of the present invention should not be limited to the described embodiments but should be defined by the claims described below as well as equivalents to these claims.

### [Industrial Applicability]

The device and method for analyzing a medical image according to an embodiment analyze a medical image by using an artificial intelligence model, and thus are applicable to the digital medical industry.

## Claims

1. A device for analyzing a medical image by using artificial intelligence, the device comprising:
one or more processors; and
a memory storing instructions executed by the one or more processors,
wherein the one or more processors are configured to: input the medical image to a first model to extract a feature vector that a second model uses to perform a first task; input the feature vector to the second model to cause the second model to perform the first task; provide a first medical result describing a health state of the medical image based on an output result of the second model; and perform a second task requested by a user of the device with respect to the first medical result,
wherein the second task includes: a first additional task of transferring the first medical result to the outside; and/or a second additional task of performing a secondary analysis including at least one of assisted decision-making and difference analysis on the first medical result.

2. The device of claim 1, wherein the medical image includes at least one of an electrocardiogram image and a chest radiographic image acquired by the device.

3. The device of claim 1, wherein the one or more processors are configured to: when a request to transfer the first medical result to the outside is received from the user, display, on a screen of the device, information of one or more other users to whom the transfer is possible, the information of the one or more other users including at least one of a department and a medical institution to which the other user belongs; and transfer the first medical result to a specific user selected by the user from among the one or more other users.

4. The device of claim 3, wherein the one or more processors are configured to: record a transfer history of the first medical result between the user and the specific user in a consultation channel in which the user and the specific user participate; and display, on a preview screen of the consultation channel, at least one of a department to which the other user belongs, the medical institution to which the other user belongs, a last conversation content of the consultation channel, and a last conversation time.

5. The device of claim 4, wherein the one or more processors are configured to display the first medical result on a background screen in the consultation channel.

6. The device of claim 1, wherein the one or more processors are configured to display, on a screen of the device, all medical results including the first medical result and another medical result previously acquired by the device, together with whether output has succeeded, in a list, wherein the other medical result includes a medical result that the device previously acquired by inputting another medical image to the first model and the second model before inputting the medical image to the first model.

7. The device of claim 6, wherein the one or more processors are configured to: analyze, for some medical results, selected by the user and including the first medical result, from among all the medical results, a difference with respect to the first medical result based on a selection order of the user; and provide the difference visually based on the selection order.

8. The device of claim 7, wherein the one or more processors are configured to display, together with a list of the some medical results, a number corresponding to the selection order of the user.

9. The device of claim 1, wherein the one or more processors are configured to provide, as the first medical result, at least one of a rhythm type of a heart, a contractility, a value and distribution of a biomarker, a sensitivity, and a health-related recommendation corresponding to the medical image.

10. The device of claim 1, wherein the one or more processors are configured to input an original image of the medical image to a third model to process the original image into the medical image, wherein the third model is pre-trained to, when an input image is input, classify an examination type of the input image and process the input image according to a region of interest that is set corresponding to the examination type.

11. The device of claim 1, wherein the one or more processors are configured to, when a click event by the user occurs on a first user interface displayed on a screen of the device, call a function linked to the click event, and through the function, remove the first user interface from the screen and display a second user interface on the screen.

12. The device of claim 11, wherein the second task includes a secondary analysis function accessible by the device through purchase in an app store in which one or more web services are registered, and the secondary analysis function is executed when a click event occurs on the second user interface.

13. A method for analyzing a medical image by using artificial intelligence performed by a device including one or more processors and a memory storing instructions executed by the one or more processors, the method comprising:
inputting the medical image to a first model to extract a feature vector that a second model uses to perform a first task;
inputting the feature vector to the second model to cause the second model to perform the first task;
providing a first medical result describing a health state of the medical image based on an output result of the second model; and
performing a second task requested by a user of the device with respect to the first medical result,
wherein the performing of the second task includes: performing a first additional task of transferring the first medical result to the outside; and/or performing a second additional task of performing a secondary analysis including at least one of assisted decision-making and difference analysis on the first medical result.

14. The method of claim 13, wherein the medical image includes at least one of an electrocardiogram image and a chest radiographic image acquired by the device.

15. The method of claim 13, wherein the performing of the first additional task includes: when a request to transfer the first medical result to the outside is received from the user, displaying, on a screen of the device, information of one or more other users to whom the transfer is possible, the information of the one or more other users including at least one of a department and a medical institution to which the other user belongs; and transferring the first medical result to a specific user selected by the user from among the one or more other users.

16. The method of claim 15, wherein the performing of the first additional task includes: recording a transfer history of the first medical result between the user and the specific user in a consultation channel in which the user and the specific user participate; and displaying, on a preview screen of the consultation channel, at least one of a department to which the other user belongs, the medical institution to which the other user belongs, a last conversation content of the consultation channel, and a last conversation time.

17. The method of claim 16, wherein the performing of the first additional task includes displaying the first medical result on a background screen in the consultation channel.

18. The method of claim 13, wherein the performing of the first additional task includes displaying, on a screen of the device, all medical results including the first medical result and another medical result previously acquired by the device, together with whether output has succeeded, in a list, wherein the other medical result includes a medical result that the device previously acquired by inputting another medical image to the first model and the second model before inputting the medical image to the first model.

19. The method of claim 18, wherein the performing of the second additional task includes: analyzing, for some medical results, selected by the user and including the first medical result, from among all the medical results, a difference with respect to the first medical result based on a selection order of the user; and providing the difference visually based on the selection order.

20. The method of claim 19, wherein the performing of the second additional task includes displaying, together with the list of the some medical results, a number corresponding to the selection order of the user.

21. The method of claim 13, wherein the providing of the first medical result includes providing, as the first medical result, at least one of a rhythm type of a heart, a contractility, a value and distribution of a biomarker, a sensitivity, and a health-related recommendation corresponding to the medical image.

22. The method of claim 13, further comprising inputting an original image of the medical image to a third model to process the original image into the medical image, wherein the third model is pre-trained to, when an input image is input, classify an examination type of the input image and process the input image according to a region of interest that is set corresponding to the examination type.

23. The method of claim 13, further comprising: when a click event by the user occurs on a first user interface displayed on a screen of the device, calling a function linked to the click event; and through the function, removing the first user interface from the screen and displaying a second user interface on the screen.

24. The method of claim 23, wherein the second task includes an additional analysis function accessible by the device through purchase in an app store in which one or more web services are registered, and the additional analysis function is executed when a click event occurs on the second user interface.
